# EUROPEAN PATENT SPECIFICATION

(11) **EP 3 669 871 B1**
(45) Date of publication and mention of the grant of the patent: **03.11.2021**
(21) Application number: 17926187.0
(22) Date of filing: 19.09.2017
(51) Int. Cl.: A61K 31/395, A61P 35/00, A61P 3/00, A61P 21/00

(54) **AMD3100 FOR THE TREATMENT AND/OR PREVENTION OF CACHEXIA, AND PHARMACEUTICAL COMPOSITION THEREOF**
AMD3100 ZUR BEHANDLUNG UND / ODER VORBEUGUNG VON KACHEXIE UND DER PHARMAZEUTISCHEN ZUSAMMENSETZUNG DAVON
AMD3100 POUR LE TRAITEMENT ET / OU LA PRÉVENTION DE LA CACHEXIE ET SA COMPOSITION PHARMACEUTIQUE

(43) Date of publication of application: 24.06.2020
(73) Proprietor: Sun Yat-Sen University, Guangzhou, Guangdong 510080 (CN)
(72) Inventor: XIANG, Peng, Guangzhou Guangdong 510080 (CN); WANG, Jiancheng, Guangzhou Guangdong 510080 (CN)
(74) Representative: Sun, Yiming
(86) International application number: PCT/CN2017/102264
(87) International publication number: WO 2019/056175

(56) References cited:
- CN-A- 104 069 061
- CN-A- 107 243 006
- D. UCHIDA ET AL: "Involvement of an Autocrine Stromal Cell Derived Factor-1/CXCR4 System on the Distant Metastasis of Human Oral Squamous Cell Carcinoma", MOLECULAR CANCER RESEARCH, vol. 5, no. 7, 1 July 2007 (2007-07-01), pages 685-694, XP055712960, US ISSN: 1541-7786, DOI: 10.1158/1541-7786.MCR-06-0368
- I. M. GHOBRIAL: "Myeloma as a model for the process of metastasis: implications for therapy", BLOOD, vol. 120, no. 1, 24 April 2012 (2012-04-24), pages 20-30, XP055476873, US ISSN: 0006-4971, DOI: 10.1182/blood-2012-01-379024
- ABDEL KAREEM AZAB ET AL: "CXCR4 inhibitor AMD3100 disrupts the interaction of multiple myeloma cells with the bone marrow microenvironment and enhances their sensitivity to therapy", BLOOD, AMERICAN SOCIETY OF HEMATOLOGY NLD, US, vol. 113, no. 18, 30 April 2009 (2009-04-30), pages 4341-4351, XP002663167, ISSN: 1528-0020, DOI: 10.1182/BLOOD-2008-10-186668 [retrieved on 2009-01-12]
- A I CHEN ET AL: "Clinical experience with a simple algorithm for plerixafor utilization in autologous stem cell mobilization", BONE MARROW TRANSPLANTATION, vol. 47, no. 12, 1 December 2012 (2012-12-01), pages 1526-1529, XP055713154, GB ISSN: 0268-3369, DOI: 10.1038/bmt.2012.74
- DE CLERCQ ET AL: "The AMD3100 story: The path to the discovery of a stem cell mobilizer (Mozobil)", BIOCHEMICAL PHARMACOLOGY, ELSEVIER, US, vol. 77, no. 11, 1 June 2009 (2009-06-01), pages 1655-1664, XP026049960, ISSN: 0006-2952, DOI: 10.1016/J.BCP.2008.12.014 [retrieved on 2008-12-31]
- CHANG Chunkang et al.: "Research progress of CXCR4 receptor blocker AMD3100", Zhongguo shiyan xueyexue zazhi = Journal of experimental hematology, vol. 19, no. 3, 31 December 2011 (2011-12-31), pages 831-834, XP009519547, CHINA ISSN: 1009-2137
- YE Huaping et al.: "Research progress of Tumor Dyscrasia", Journal of Military Surgeon in Southwest China, vol. 16, no. 3, 31 May 2014 (2014-05-31), pages 302-304, XP009519522, ISSN: 1672-7193
- MARTINELLI, G. B.: "Activation of the SDF1/CXCR4 pathway retards muscle at- rophy during cancer cachexia", Oncogene, vol. 35, no. 48, 23 May 2016 (2016-05-23), - 1 December 2016 (2016-12-01), pages 6212-6222, XP055584539, ISSN: 0950-9232, DOI: 10.1038/onc.2016.153

### SEQUENCE LISTING

<110> ÖÐÉ½'óÑ§
<120> AMD3100ÔÚÖÆ±,ÖÎÁÆ°Í/>>òÔ¤•À¶ñÒ°ÖÊµÄÒⒸÎïÖÐµÄÓ¦ÓÃ¼°ÆäÒⒸÎï×é°ÏÎï
<130> 2017
<160> 26
<170> PatentIn version 3.3
<210> 1
   <211> 23
   <212> DNA
   <213> ÈË¹¤ÐòÁÐ
<400> 1
   ggctacatac aggattctgc tgg 23
<210> 2
   <211> 21
   <212> DNA
   <213> ÈË¹¤ÐòÁÐ
<400> 2
   caggaaagcc aagagaagcc t 21
<210> 3
   <211> 23
   <212> DNA
   <213> ÈË¹¤ÐòÁÐ
<400> 3
   ggagctgcac acaacccatt gcc 23
<210> 4
   <211> 23
   <212> DNA
   <213> ÈË¹¤ÐòÁÐ
<400> 4
   gatcactctc ggcatggacg age 23
<210> 5
   <211> 21
   <212> DNA
   <213> ÈË¹¤ÐòÁÐ
<400> 5
   tgtcatgctt ctgggcctgc t 21
<210> 6
   <211> 21
   <212> DNA
   <213> ÈË¹¤ÐòÁÐ
<400> 6
   ttcactgtca cactggtcac t 21
<210> 7
   <211> 18
   <212> DNA
   <213> ÈË¹¤ÐòÁÐ
<400> 7
   ggtctccaca ctgccctt 18
<210> 8
   <211> 21
   <212> DNA
   <213> ÈË¹¤ÐòÁÐ
<400> 8
   tcaggcattc agttccaggt c 21
<210> 9
   <211> 20
   <212> DNA
   <213> ÈË¹¤ÐòÁÐ
<400> 9
   atatggctcg gacaccactc 20
<210> 10
   <211> 20
   <212> DNA
   <213> ÈË¹¤ÐòÁÐ
<400> 10
   tccttcgagt gacaaacacg 20
<210> 11
   <211> 20
   <212> DNA
   <213> ÈË¹¤ÐòÁÐ
<400> 11
   gtgtccctgg gaagctgtta 20
<210> 12
   <211> 20
   <212> DNA
   <213> ÈË¹¤ÐòÁÐ
<400> 12
   ctttggagtt ggggttttca 20
<210> 13
   <211> 19
   <212> DNA
   <213> ÈË¹¤ÐòÁÐ
<400> 13
   cgcagtgctt ctttgcctg 19
<210> 14
   <211> 20
   <212> DNA
   <213> ÈË¹¤ÐòÁÐ
<400> 14
   tctggcccag tcagcttctc 20
<210> 15
   <211> 20
   <212> DNA
   <213> ÈË¹¤ÐòÁÐ
<400> 15
   gctgggattc acctcaagaa 20
<210> 16
   <211> 20
   <212> DNA
   <213> ÈË¹¤ÐòÁÐ
<400> 16
   aagggagctt cagggtcaag 20
<210> 17
   <211> 20
   <212> DNA
   <213> ÈË¹¤ÐòÁÐ
<400> 17
   gccaagggtt gacttcaaga 20
<210> 18
   <211> 20
   <212> DNA
   <213> ÈË¹¤ÐòÁÐ
<400> 18
   ttcagggtca aggcaaactt 20
<210> 19
   <211> 22
   <212> DNA
   <213> ÈË¹¤ÐòÁÐ
<400> 19
   ttctaaatca gagaaaagcg at 22
<210> 20
   <211> 21
   <212> DNA
   <213> ÈË¹¤ÐòÁÐ
<400> 20
   tagatgcaat tatacccgta g 21
<210> 21
   <211> 22
   <212> DNA
   <213> ÈË¹¤ÐòÁÐ
<400> 21
   tgtaatttac ccgagtaacg gc 22
<210> 22
   <211> 23
   <212> DNA
   <213> ÈË¹¤ÐòÁÐ
<400> 22
   cacctttgtc gtttatgagc ctt 23
<210> 23
   <211> 21
   <212> DNA
   <213> ÈË¹¤ÐòÁÐ
<400> 23
   tgcatcagtg acggtaaacc a 21
<210> 24
   <211> 21
   <212> DNA
   <213> ÈË¹¤ÐòÁÐ
<400> 24
   ttcttcagcc gtgcaacaat c 21
<210> 25
   <211> 20
   <212> DNA
   <213> ÈË¹¤ÐòÁÐ
<400> 25
   tcttgccaga aagatatccc 20
<210> 26
   <211> 21
   <212> DNA
   <213> ÈË¹¤ÐòÁÐ
<400> 26
   ttttaattgc acaatactcc c 21

## Description

### TECHNICAL FIELD

The disclosure relates to the field of medicines, and particularly to application of AMD3100 or a pharmaceutically acceptable salt thereof for treatment and/or prevention of cachexia and a pharmaceutical composition thereof for treating and/or treating cachexia

### BACKGROUND

Tumor cachexia is a common concomitant symptom in cancer patients, and is mainly manifested as involuntary weight loss and anorexia. At least 30% of cancer patients die of cachexia, and at least 50% of cancer patients die with cachexia. The definition of tumor cachexia, drafted by Professor Fearon of School of clinical science and community health, University of Edinburgh, UK, has basically reached a consensus at present: cachexia is a multifactorial syndrome, which is manifested as a fact that a patient is losing the mass of skeletal muscles (with or without loss of fat mass), while the traditional nutritional support can not completely reverse it, and then causes continuous deterioration of functional damage. The tumor cachexia not only reduces the life quality of patients, but also leads to the tolerance of tumor treatment and weakens the effect of tumor treatment. Therefore, treatment for tumor cachexia plays an important role in treating tumor and improving the life quality of patients.

With the development of a large amount of researches, the pathogenesis of tumor cachexia has been further deeply understood. The pathophysiological feature of tumor cachexia is that protein and energy are in negative balance which is caused by reduced food intake and abnormal metabolism integrated factors. Although many preclinical studies have gained good progress in an animal model having tumor cachexia at present, there are still no drugs to be granted to be applied to prevention and treatment of tumor cachexia in clinic. Clinically, occurrence of tumor cachexia is delayed mainly through nutrition support and exercises.

Thus, it is urgent to deeply know the pathogenesis of tumor cachexia Targeted intervene is carried out at the early stage of tumor cachexia in order to achieve better therapeutic effect.

The mesenchymal stem cell (MSC) is a non-hematopoietic stem cell (Friedenstein, A.J., et al, 1974) found in bone marrow at the earliest, which participates in forming the hematopoietic microenvironment of bone marrow and has an obvious support effect on the proliferation and differentiation of hematopoietic stem cells. MSC is widely distributed in various tissues and organs of the whole body. In addition to bone marrow, it also exists in gingiva, skeletal muscles, fats and other tissues to participate in damage repair and homeostasis maintenance of tissues. MSC is in lack of specific markers, mainly expressing mesenchymal markers such as CD29, CD44, CD73, CD90, CD105 and CD166, rather than hematopoiesis-related markers such as CD11b, CD14, CD19, CD34 and CD45.

Nestin is an intermediate silk protein. At the stage of embryonic development, Nestin is first expressed in neural epithelial stem cells having multi-directional differentiation potential. However, in adult tissues, the intermediate silk protein Nestin is only expressed in adult stem/progenitor cell population that is not fully differentiated and maintains a certain proliferative ability. Therefore, such the Nestin+ cell is an important component of an adult stem cell pool and plays an important role in maintaining the homeostasis of stem cells of a body. Recently, Mendez Ferrer et al. firstly confirm that a group of Nestin+ cells are present in bone marrow by using Nestin-GFP transgenic mice, and can be cloned and grow in vitro and have the characteristics of osteogenic, lipogenic and chondrogenic differentiation, suggesting that the intermediate silk protein Nestin is an important marker of a mesenchymal stem cell (MSC) of bone marrow. In the early stage of the present application, the research of the inventor using the Nestin-GFP transgenic mice shows that GFP positive cells isolated from testis, kidney, heart and other tissues have the potential of self-renewal and multi-directional differentiation, and have the ability of repairing damaged tissues. These results indicate that Nestin+ MSC plays an important role in maintaining tissue homeostasis.

In summary, adult stem cells have the functions of self-renewal, replacement or repair of damaged tissues accompanying with the lives of different organs. As a member of adult stem cells, MSC is an important component for maintaining the homeostasis of the interiors of the body tissues. In the process of tumor development, tumor has the ability of recruiting Nestin+ MSC to enter tumor microenvironment. After Nestin+ MSC is recruited by tumors, tissue homeostasis is destroyed, which causes the dysfunction of organ tissues and becomes an important factor to induce tumor cachexia.

MSC expresses multiple chemokine and adhesion molecule receptors. In the presence of ligands, MSC can be mediated to migrate toward injury or inflammation parts. It is reported for many times that MSC is recruited to enter tumor microenvironment in the course of tumor development. In 2011, Michael quante and his colleagues found that in the mouse model with chronic gastric cancer induced by Helicobacter pylori, the diseased tissue recruited Nestin+ cells from the bone marrow; in 2014, Diana Klein and his colleagues found that mouse B16F10 and LLC tumors recruited Nestin+ cells of tissues except bone marrow to participate in the formation of blood vessels. These results suggest that tumors have the ability of recruiting MSC of the body with Nestin as a marker. However, the mechanism of recruiting MSC by tumors has not been fully and clearly studied.

AMD3100 has the chemical name 1,1'-[1,4-phenylene bis(methylene)]-di-1,4,8,11-tetraazacyclotetradecane. Its structural formula is as follows:

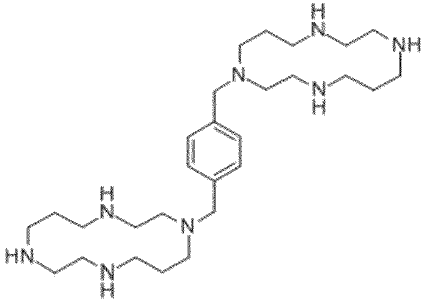

AMD3100 was first synthesized in 1994, and its therapeutic potential is known to include HIV infection, inflammatory diseases, stem cell mobilization, leukemia and solid tumors. So far, there have not been reports on AMD3100 in prevention and treatment of cachexia.

D. UCHIDA ET AL: Molecular Cancer Research, Vol.5(7), 2007, pages 685-694, discloses that the treatment with AMD3100 significantly ameliorated the body weight loss of tumor bearing mice.

### SUMMARY

Through long-term research of the inventor of the disclosure, compound 1,1'-[1,4-phenylene bis(methylene)]-di-1,4,8,11-tetraazacyclotetradecane or a pharmaceutically acceptable salt thereof can effectively treat and/or prevent cachexia, especially, provides a new thinking for treatment of tumor cachexia, which is beneficial to delaying or relieving the cachexia symptoms of tumor patients, improving the treatment effect of tumors, improving the life's quality and prolonging the survival expectation.

The invention provides the compound 1,1'-[1,4-phenylene bis(methylene)]-di-1,4,8,11-tetraazacyclotetradecane or a pharmaceutically acceptable salt thereof for use in a method of relieving and/or improving fat loss caused by cachexia.

Another aspect of the invention provides a pharmaceutical composition for use in a method of relieving and/ or improving fat loss caused by cachexia, the pharmaceutical composition comprising the compound 1,1'-[1,4-phenylene bis(methylene)]-di-1,4,8,11-tetraazacyclotetradecane or the pharmaceutically acceptable salt thereof.

Preferably, the pharmaceutical composition also contains at least one of other drugs. The drug or pharmaceutical composition of the disclosure can be used in treatment and/or prevention of cachexia in mammals (for example human, mice, rats, rabbits, dogs, cats, cows, horses, pigs and monkeys).

The subject of the disclosure can refer to any animals, including but not limited to human, mice, rats, rabbits, dogs, cats, cows, horses, pigs and monkeys and other mammals. The method of the disclosure is similarly applicable to combination with a certain effective dosage of other drugs or pharmaceutical compositions to synergistically act on treatment and/or prevention of cachexia.

The drug or pharmaceutical composition of the disclosure can be applied via any physiologically acceptable routes, such as oral administration and injection. In embodiments provided by the disclosure, the dosage form of AMD3100 is injection.

When use in the above treatment and/or prevention or other treatments and/or preventions, the total daily amount of the compound and composition of the disclosure must be decided by the chief physician within the range of reliable medical judgment. For any specific patients, the specific therapeutic effective dose level must be determined depending on multiple factors which include the treated obstacle and the severity of the obstacle; the activity of the used specific compound; the used specific composition; the age, weight, general health status, gender and diet of the patient; the administration time, administration route and excretion rate of the used specific compound; treatment duration; drugs used in combination with or at the same time with the used specific compounds; and similar factors known in the medical field. For example, the practice in the art is that the dosage of the compound starts from lower than a level required for obtaining the needed therapeutic effect, and the dosage is gradually increased until the required effect is obtained.

The drug or pharmaceutical composition of the disclosure can be prepared into pharmaceutically acceptable dosage forms, in general, the effective ingredients of the drug account for 1-95% of the total mass of the administrated therapeutic drug. The dosage form of the drug needs to be applicable to different administration ways, such as oral preparations (such as tablets, capsules, solutions or suspensions); injectable preparations (such as injectable solutions or suspensions, or injectable dry powder which can be dissolved or dispersed into sterile water or other sterile injectable media prior to use). These preparations can be prepared by adopting the traditional process.

The drug or pharmaceutical composition of the disclosure can also contain pharmaceutically allowable excipients or carriers. Pharmaceutically acceptable excipients or carriers can be selected as needed. These excipients or carriers are well known, including: adhesives used for oral preparations (such as starch, gelatin, methylcellulose, sodium carboxymethylcellulose and/or polyvinylpyrrolidone), diluents (such as lactose, dextrose, sucrose, mannitol, sorbitol, cellulose and/or glycerin), lubricants (such as silica, talc, stearic acid or its salts, usually magnesium stearate or calcium stearate, and/or polyethylene glycol), and if required, it also contains disintegrating agents, such as starch, agar, alginic acid or its salts, usually sodium alginate, and/or effervescent mixture, cosolvents, stabilizers, suspending agents, colorless pigment and flavoring agents, preservatives, solvents, stabilizers and the like used for injectable preparations; matrix, diluents, lubricants, preservatives and the like used for local preparations.

"Pharmaceutically allowable" means that the adjuvant or carrier is compatible with the active ingredients, optimally, can stabilize the active ingredients and is harmless to the treated individual.

The cachexia of the disclosure includes chronic diseases such as malignant tumors, tuberculosis, diabetes, blood diseases, endocrine diseases, infectious diseases, acquired immune deficiency syndrome and the like, and systemic syndromes with significant weight loss, anorexia and the like as main symptoms. For example, cancer cachexia, tuberculosis cachexia, diabetes cachexia, blood disease cachexia, endocrine disease cachexia, infectious cachexia, and cachexia caused by acquired immune deficiency syndrome.

Preferably, the cachexia is tumor cachexia (namely, cachexia caused by tumors).

Preferably, the treatment and/or prevention of cachexia comprises relieving or improving the symptoms of tumor cachexia.

According to the invention, the treatment and/or prevention of cachexia comprises relieving or improving cachexia symptoms of fat loss.

Treatment and/or prevention of cachexia used herein comprises relieving or improving the cachexia symptom of a patient, namely fat loss.

The phrase "pharmaceutically acceptable salt" used herein refers to some salts that can maintain their original biological activity and are suitable for medical use. The compound 1,1'-[1,4-phenylene bis(methylene)]-di-1,4,8,11-tetraazacyclotetradecane can produce many different salts with different inorganic and organic acids. The acids for preparing the salts can be acids for generating nontoxic acid addition salts, including but not limited to citrate, maleate, hydrochloride, hydrobromate, hydroiodate, nitrate Sulfate, bisulfate, phosphate, acetate, lactate, salicylate, citrate, succinate, maleate, gentianate, fumarate, formate, benzoate, methanesulfonate, ethanesulfonate and benzenesulfonate. Preferably, the salt is selected from hydrobromate and hydrochloride.

The term "treatment" used herein refers to reliving or improving diseases, namely, delaying or inhibiting the development of the diseases or at least one clinical symptom. The term "prevention" used herein refers to administering the compound of the disclosure to a subject prior to the occurrence of the symptoms of the disease.

As used herein, the subjects "need" such the treatment if they biologically, medically or qualitatively benefit from treatment.

The inventor of the present application proposes that the deficiency of mesenchymal stem cells of the body is a reason why tumor cachexia occurs. After long-term research, it is found that the compound 1,1'-[1,4-phenylene bis(methylene)]-di-1,4,8,11-tetraazacyclotetradecane or the pharmaceutically acceptable salt thereof can effectively treat and/or prevent cachexia provides a new thinking for treatment of cachexia They mainly block the recruitment of tumors on mesenchymal stem cells of multiple systemic tissues, thereby being conducive to maintaining the homeostasis of the body, delaying the occurrence of cachexia reducing or alleviating the symptoms of cachexia, improving the therapeutic effect, improving the quality of life and prolonging the survival expectancy of patients.

### BRIEF DESCRIPTION OF THE DRAWINGS

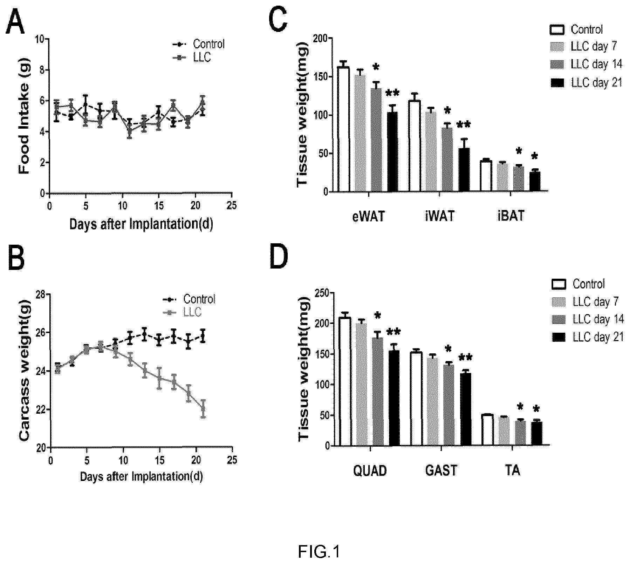

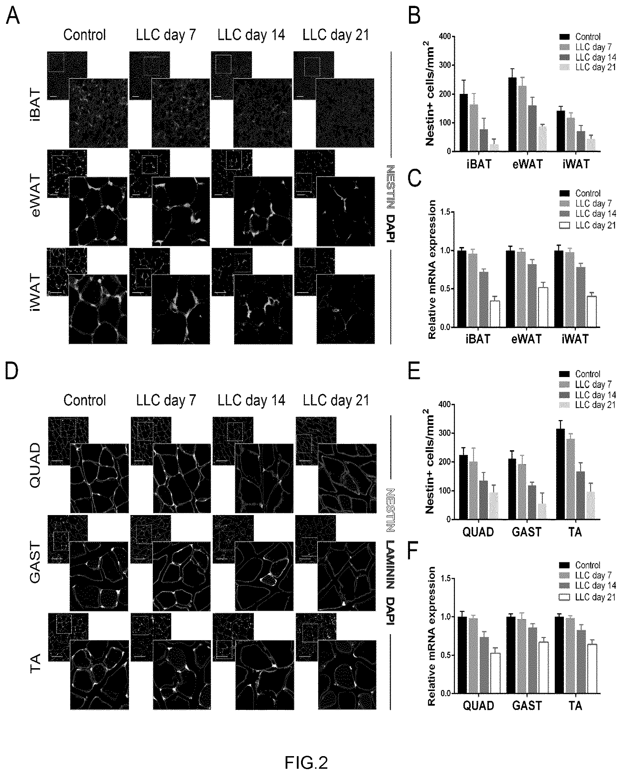

Fig. A-D in Fig. 1 are respectively mice in C57 BL/6 cachexia group after subcutaneous transplantation of LLC tumor cells and control group: (A) mouse feeding detection; (b) mouse weight monitoring; (C) and (D) weight detection of fat tissues and muscle tissues respectively.
Fig. A-D in Fig. 2 are control group and cachexia group of Nestin-GFP transgenic mice respectively: (A) and (B) are immunofluorescence staining of mouse fat tissue sections and statistics of Nestin-GFP+ cells in the sections; (C) is fluorescence quantitative PCR detection on expression of Nestin in fat tissues; (D) and (E) are immunofluorescence staining of mouse muscle tissue sections and statistics of Nestin-GFP+ cells in the sections, respectively; (F) is fluorescence quantitative PCR detection on expression of Nestin in muscle tissues.
Fig. 3 shows that after subcutaneous transplantation of LLC tumor cells in Nestin-GFP transgenic mice: (A) is immunofluorescence staining of mouse tumor tissue sections and statistics of Nestin-GFP+ cells in the sections; (B) is fluorescence quantitative PCR detection of GFP expression in tumor tissues; (C) and (D) are flow cytometry detection of Nestin-GFP+ cells in peripheral blood.
Fig. 4 shows quantitative PCR detection result of tumor chemokine expression.
Fig. 5 shows detection results of Nestin positive cell expression chemokine receptor in muscles and fat tissues of mice.
Fig. 6 shows experimental results of promoting the migration of mouse Nestin+ cells by Cxcl 12.
Fig. 7 shows experimental results of in vivo inhibition of Nestin+ cell migration by AMD3100.
Fig.8 shows monitoring results of mouse tumor dyscrasia symptoms after AMD3100 is used in vivo.
Fig.9 shows experimental results of lifetime of tumor-bearing mice after AMD3100 is used in vivo.

### DESCRIPTION OF THE EMBODIMENTS

The technical solution of the disclosure will be further described in combination with accompanying drawings below.

AMD3100 was purchased from American ApexBio company, and its item number is A2025.

Unless otherwise noted, all reagents used in examples are conventional reagents existing in the art and can be commercially purchased. All experimental operations that are not specially illustrated in examples are conventional operations in the art or can be understood or known by those skilled in the art according to the grasped existing technologies or well-known knowledge.

### Example 1 Culture of Lewis lung cancer LLC and isolation and culture of mouse Nestin+ cells

### 1.1 LLC cell culture: LLC cells (ATCC #CRL-1642) were cultured using a DMEM basic culture medium (containing 10% fetal bovine serum) for later use.

### 1.2 Isolation of mouse muscle Nestin+ cells

The adult Nestin-GFP transgenic male mice (provided by Dr Masahiro Yamaguchi, also commercially available) were taken, and gastrocnemius muscles at both sides were completely cut under sterile conditions and quickly placed in a culture dish containing PBS. The muscle blocks were rinsed 3 times with PBS and then cut into small muscle tissues, and the blood vessels, nerves and connective tissues were removed as much as possible. Muscle cells were digested and isolated by using type I collagenase: the muscle tissues were transferred to a centrifuge tube, added with type I collagenase and digested for 30min in 37°C constant-temperature oscillation water bath box. Serum was added to stop digestion, the digestion solution was centrifuged at 1100rpm for 4 minutes, the supernatant was sucked, and the precipitate was re-suspended with PBS and filtered through a 200-mesh filter screen. The filtrate was collected and centrifuged for 4min, the supernatant was removed, and the precipitate was re-suspended with PBS. Nestin+ cells were sorted by flow cytometry.

### 1.3 Isolation of mouse fat Nestin+ cells

About 6-week-old male Nestin-GFP transgenic mice were selected. The fat tissues in the groins of the mice were isolated under sterilized condition. After washing with PBS (at least 6 times), the fat tissues were sufficiently cut into pieces with scissors. 10ml of 0.1% collagenase IV + 0.05% pancreatin + 0.1% Dispase II mixed solution was added and stirred for 60min at 37°C. Then the α-MEM culture medium containing 10% FBS was added to terminate the digestion. The digested solution was filtered with 100-mesh and 200-mesh screens, centrifuged for 5min at 800g, the supernatant was discarded, the culture medium was added, and the cell suspension was prepared by gently beating and evenly mixing; Nestin+ cells were sorted by flow cytometry.

### 1.4 Culture of mouse muscle and fat-derived Nestin+ cells

The Nestin+ cells obtained from muscle and fat tissues in 1.2 and 1.3 were subjected to adherent culture with DMEM/low sugar (10% fetal bovine serum) culture medium.

### Example 2 Feeding of mice and construction of tumor cachexia model

2.1 Feeding method of mice: mice were fed with conventional mouse fodder (the content of protein was 20~25wt%, the content of fat was 5%~10wt%, and the content of crude fiber was 3-5wt%).
2.2 Construction of mouse tumor cachexia model 8-week-old male C57 BL/6 mice (purchased from Nanjing University Institute of model animals) or Nestin-GFP transgenic mice having no more than 2g of weight were taken and randomly divided into control group and cachexia group. About 4 × 10⁶ Lewis lung cancer LLC cells were injected subcutaneously into the groin of each mouse in cachexia group. The experimental process was carried out in an ultra-clean platform. Attention was paid to aseptic operation. The day of inoculation was day 0, and the next day was day 1, day 2.... in sequence. On the day7 of tumor inoculation, it was detected that the weight of tumor-bearing mice began to decrease, and the tumor cachexia had began to occur; on the day 21, the weights of mice were significantly decreased, and the cachexia sign was obvious.

### Example 3: Evaluation of mouse tumor cachexia

In example 2, after the mice in cachexia group were inoculated with tumor, the hair color, activity, weight, food intake and tumor size of mice were observed at the same time every day, and compared with those in control group. After tumor inoculation, mice were killed using excessive anesthetization every 2 days until the day 21, or on the day 7, day 14 and day 21. The tumor tissues were removed and the weights of all animals without tumors were weighed. Quadriceps femoris (QUAD), gastrocnemius (GAST), tibialis anterior (TA), epididymis fat tissue (eWAT), inguinal fat tissue (iWAT), brown fat tissues between dorsal and scapular (iBAT) were isolated, and weighed respectively.

In Fig.1, Fig. A shows daily eating test results of C57 BL/6 mice in control group and cachexia group, Fig. B shows weight monitoring results of control group and cachexia group, and Fig. C and Fig. D show weight detection of fat tissues and muscle tissues respectively. It can be seen from Fig. 1 that after subcutaneous transplantation of LLC tumor cells in C57 BL/6 mice, tumor dyscrasia occurred.

### Example 4: Detection of Nestin-GFP+ cells in skeletal muscles, fat tissues and tumors of mice

### 4.1 Immunofluorescence staining

Quadriceps femoris (QUAD), gastrocnemius (GAST) and tibialis anterior muscle (TA) were respectively isolated from Nestin-GFP transgenic mice in control group and cachexia group, immobilized for 8h with 4% PFA, dehydrated for 48h with 30% (mass) sucrose, and frozen and sliced. After the substrate membrane was labeled with Laminin antibody, the fluorescent second antibody was incubated, the cell nucleus was re-stained for 5min with 0.2% 1g/mL DAPI, and the tissues were washed twice with 0.01M PBS and observed under the microscope.

Epididymal fat tissues (eWAT), inguinal fat tissues (iWAT) and interscapular brown fat tissues (iBAT) were respectively isolated from C57 BL/6 mice in control group and cachexia group, immobilized for 8h with 4% PFA, underwent gradient dehydration with alcohol, and sliced with paraffin in turn. After gradient dewaxing with xylene and alcohol, the above sliced tissues were subjected to antibody staining with Nestin by reference to the above skeletal muscle staining method, and observed under the microscope.

The treatment of tumor tissues of Nestin-GFP transgenic mice in cachexia group was identical to that of muscle tissues. After being frozen and sliced, the cell nuclei were re-stained with 0.2% 1g/mL DAPI for 5min, and the tumor tissues were washed twice with 0.01M PBS and observed under the microscope.

Fig. A in Fig. 2 shows observation results of immunofluorescence staining of mouse muscle tissue sections of Nestin-GFP transgenic mouse dyscrasia group on the day 7, day 14 and day 21 after subcutaneous transplantation of LLC tumor cells and control group; FIG. B in Fig. 2 shows statistic results of Nestin-GFP+ cells in mouse muscle tissue sections of Nestin-GFP transgenic mouse cachexia group on the day 7, day 14 and day 21 after subcutaneous transplantation of LLC tumor cells and control group.

Fig. D in Fig. 2 shows observation results of immunofluorescence staining of mouse muscle tissue sections of Nestin-GFP transgenic mouse cachexia group on the day 7, day 14 and day 21 after subcutaneous transplantation of LLC tumor cells and control group cultured for 21 days; Fig. E in Fig. 2 shows statistic results of Nestin-GFP+ cells in mouse muscle tissue sections of Nestin-GFP transgenic mouse cachexia group on the day 7, day 14 and day 21 after subcutaneous transplantation of LLC tumor cells and control group cultured for 21 days.

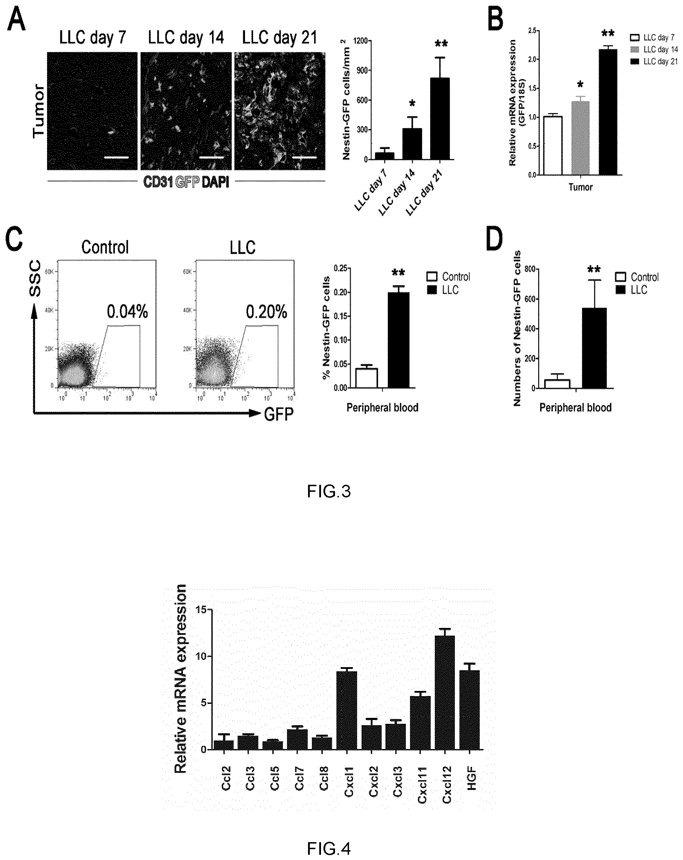

Fig. A in Fig. 3 shows observation results of immunofluorescence staining of tumor tissue sections of Nestin-GFP transgenic mouse cachexia group on the day 7, day 14 and day 21 after subcutaneous transplantation of LLC tumor cells; Fig. C and D in Fig. 3 are result graphs of Nestin-GFP+ cells in mouse peripheral blood in control group and cachexia group detected by flow cytometry.

### 4.2 Fluorescence quantitative PCR

The specific steps of analyzing Nestin gene expression using quantitative PCR are as follows: muscles (QUAD, GAST, TA) and fats (eWAT, iWAT, iBAT) were taken from mice of each experimental group in example 2, and tumor tissues were taken from mice of cachexia group. After the tissues were ground by liquid nitrogen, total RNA was extracted by Trizol method. Reverse transcription was carried out on RNA to obtain single-stranded cDNA, and fluorescence quantitative PCR was carried out using the LC480 system of Roche company to detect the Nestin or GFP gene expression. The used Nestin primers were 5'-GGCTACATACAGGATTCTGCTGG-3' (F) and 5'-CAGGAAAGCCAAGAGAAGCCT -3' (R), and the used GFP primers were 5'- GGA GCT GCA CAC AAC CCA TTGCC -3' (F) and 5'-GAT CAC TCT CGG CAT GGA CGAGC-3 '(F). Detection results are seen in Figs. C and F in Fig. 2. Where, Fig. C in Fig. 2 shows the expression of Nestin in fat tissues in fat tissues detected by fluorescence quantitative PCR after subcutaneous transplantation of LLC tumor cells was carried out on Nestin-GFP transgenic mice; Fig. F in Fig. 2 is expression of Nestin in muscle tissues detected by fluorescence quantitative PCR after subcutaneous transplantation of LLC tumor cells was carried out on Nestin GFP transgenic mice.

Fig. B in Fig. 3 is expression of GFP in tumor tissues detected by fluorescence quantitative PCR after subcutaneous transplantation of LLC tumor cells was carried out on Nestin-GFP transgenic mice.

It can be seen from the detection results of example 4 that after subcutaneous transplantation of LLC tumor cells was carried out on Nestin-GFP transgenic mice, Nestin-GFP+ cells in skeletal muscles and fat tissues were continuously decreased and Nestin-GFP+ cells in tumor were continuously increased.

### Example 5 Detection of tumor chemokine expression and detection of mouse MSC chemokine receptor expression

### 5.1 Detection of tumor chemokine expression by fluorescence quantitative PCR

The single-stranded cDNA was obtained by the tumor tissue treatment method mentioned in 4.2, and then fluorescence quantitative PCR was carried out to expression of each chemokine gene using the Roche LC480 system.

Various primers used in fluorescence quantitative PCR are as follows:
(1) Mouse Ccl2 primers:
   5'- TGTCATGCTTCTGGGCCTGCT -3' (F) and 5'-TTCACTGTCACACTGGTCACT -3' (R);
(2) Mouse Ccl 3 primers:
   5'- GGTCTCCACACTGCCCTT -3' (F) and 5'- TCAGGCATTCAGTTCCAGGTC -3' (R);
(3) Ccl 5 primers :
   5'- ATATGGCTCGGACACCACTC -3' (F) and 5'- TCCTTCGAGTGACAAACACG -3' (R);
(4) Ccl7 primers:
   5'- GTGTCCCTGGGAAGCTGTTA -3' (F) and 5'- CTTTGGAGTTGGGGTTTTCA -3' (R);
(5) Ccl8 primers:
   5'- CGCAGTGCTTCTTTGCCTG -3' (F) and 5'- TCTGGCCCAGTCAGCTTCTC -3' (R);
(6) Cxcl1 primers:
   5'- GCTGGGATTCACCTCAAGAA -3' (F) and 5'- AAGGGAGCTTCAGGGTCAAG -3' (R);
(7) Cxcl2 primers:
   5'- GCCAAGGGTTGACTTCAAGA -3' (F) and 5'- TTCAGGGTCAAGGCAAACTT -3' (R);
(8) Cxcl3 primers:
   5'- TTCTAAATCAGAGAAAAGCGAT -3' (F) and 5'- TAGATGCAATTATACCCGTAG -3' (R);
(9) Cxcl11 primers:
   5'- TGTAATTTACCCGAGTAACGGC -3' (F) and 5'- CACCTTTGTCGTTTATGAGCCTT - 3' (R);
(10) Cxcl12 primers:
   5'- TGCATCAGTGACGGTAAACCA -3' (F) and 5'- TTCTTCAGCCGTGCAACAATC -3' (R);
(11) HGF primers:
   5'- TCTTGCCAGAAAGATATCCC -3' (F) and 5'- TTTTAATTGCACAATACTCCC -3' (R).

The results of fluorescence quantitative PCR are shown in Fig.4. It can be seen from Fig.4 that chemokine Cxcl12 is highly expressed in mouse tumor.

### 5.2 Detection of mouse MSC chemokine receptor Cxcr4 expression

The mouse fat and muscle-derived cells were immobilized for 15min with 4% PFA, then penetrated for 15min with 0.2% Triton, and then sealed for 30min with 1% BSA. The Nestin antibody (mouse source) diluted with 1:100 and the Cxcr4 antibody (rabbit source) diluted with 1:100 were incubated at 4°C for 14h. The antibodies were washed for 10min with PBS with three times, and then the green mouse fluorescent second antibody (1:500) and the red rabbit fluorescent second antibody (1:500) were incubated at 37°C for 30min. The antibodies were washed 10min with PBS with three times, the nucleus was re-stained with 0.2% 1g/mL DAPI for 5min, and the antibodies were washed twice with 0.01M PBS and observed under the microscope. It can be seen that Nestin+ cells isolated from muscle and fat tissues of mice express Cxcr4.

### Example 6: Experiment for promoting the migration of mouse Nestin+ cells by Cxcl 12

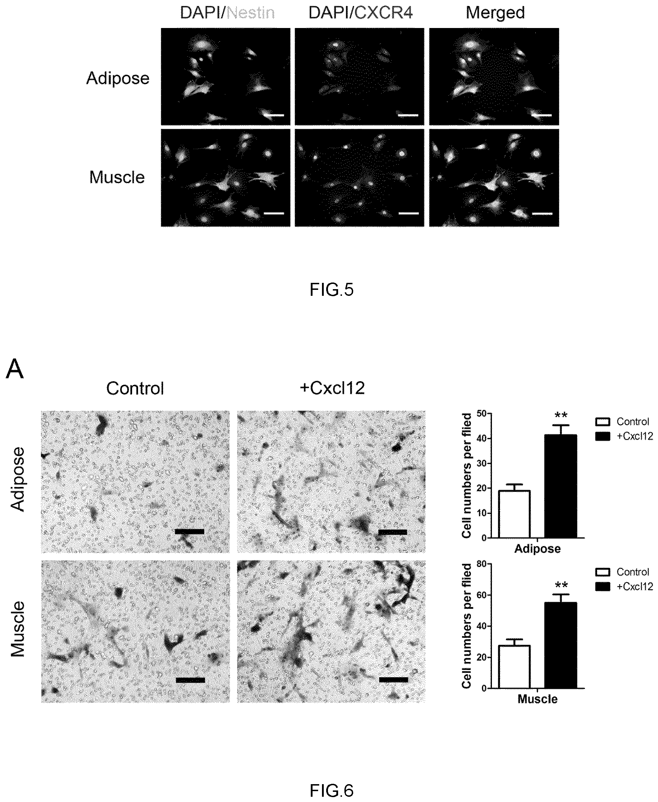

The Corning Transwell chamber (8 µm pore size) was placed on a 24-well plate, and muscle and fat-derived Nestin+ cells (see example 1) cultured in vitro were inoculated in the Transwell chamber with 2×10⁴ cells/well, and cultured in a 5% CO₂ and 37°C incubator. Each well was added with 500 µl of DMEM/low sugar under the Transwell chamber. The experimental group contained 1 µmol/ml Cxcl12 factors, while Cxcl12 factors were not added in control group. After 24 hours, the chamber was taken out, the cells inside the chamber were wiped out with a cotton swab, and the chamber was washed gently and immobilized and rinsed twice with anhydrous alcohol; the chambers in various groups were marked, and erected on the abandoned well plate, and crystal violet dye solution was added into each well for incubate at room temperature for 15min; excessive staining solution was washed away. The quantity of migrated cells in each group was measured under the inverted microscope. Five fields (400 times) were randomly selected to calculate the quantity of migrated cells in each group under the microscope with 3 repeated wells in each group. The average value of the results was calculated. The results are shown in Fig. 6. It can be seen from Fig.6 that Cxcl 12 can promote the migration of mouse Nestin+ cells.

### Example 7 Experiment for in vivo inhibition of Nestin+ cell migration by AMD3100

8-week-old C57 BL/6 male mice having no more than 2g of weight were taken and divided into control group, cachexia group (LLC), and AMD3100 group (LLC + AMD3100). Control group: no treatment. Cachexia group: 4 × 10⁶ LLC was transplanted subcutaneously into the groin of mice. After 7 days, mice were anesthetized with 100 µL of 10% chloral hydrate, and Alzet slow release pumps (model: 1004) were implanted subcutaneously on the back of mice. The normal saline was contained in the pump.

AMD3100 group: 4 × 10⁶ LLC was transplanted subcutaneously into the groin of mice. After 7 days, mice were anesthetized with 100 µL of 10% chloral hydrate, and an Alzet slow-release pump (model: 1004) was implanted subcutaneously on its back, and AMD3100 was contained in the pump. The use amount of mice AMD3100 was 0.15mg/kg weight/day. After 14 days of installation of the slow-release pump, the mice were killed, and the muscle and fat tissue sections were taken for immunofluorescence staining, or RNA reverse transcription for fluorescence quantitative PCR was carried out by extracting RNA reverse transcription to detect the expression of Nestin.

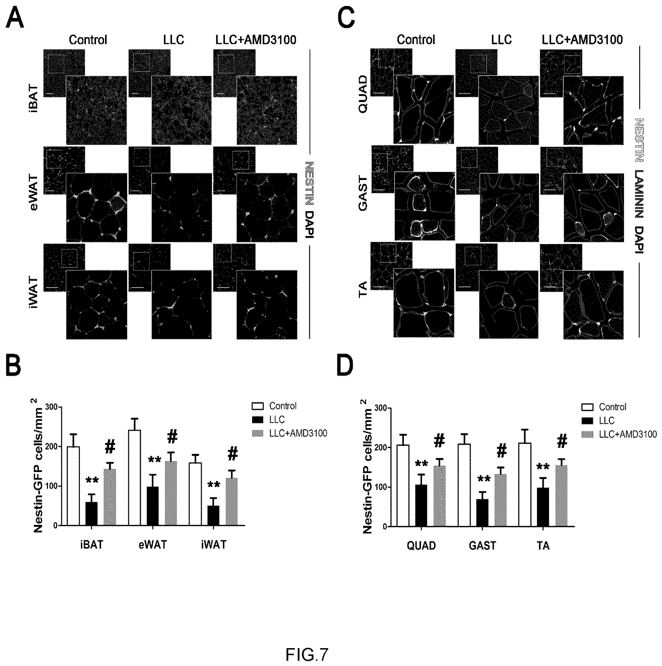

Immunofluorescence staining results are seen in Fig. A and B in Fig. 7. Fig. A in Fig. 7 shows observation results of immunofluorescence staining on mouse fat tissue sections of Nestin-GFP transgenic mouse cachexia group and AMD3100 group 21 days after subcutaneous transplantation of LLC tumor cells and control group cultured for 21 days; FIG. B in Fig. 7 shows statistic results of Nestin-GFP+ cells in mouse fat tissue sections of Nestin-GFP transgenic mouse cachexia group and AMD3100 group 21 days after subcutaneous transplantation of LLC tumor cells and control group cultured for 21 days. Fig. C and Fig. D in Fig. 7. Fig. C in Fig. 7 shows observation results of immunofluorescence staining on mouse fat tissue sections of Nestin-GFP transgenic mouse cachexia group and AMD3100 group 21 days after subcutaneous transplantation of LLC tumor cells and control group cultured for 21 days; FIG. D in Fig. 7 shows statistic results of Nestin-GFP+ cells in mouse fat tissue sections of Nestin-GFP transgenic mouse cachexia group and AMD3100 group 21 days after subcutaneous transplantation of LLC tumor cells and control group cultured for 21 days.

It can be seen from the experimental results that Nestin-GFP+ cells in skeletal muscles and fat tissues of tumor-bearing mice increased after AMD3100 was used in vivo.

### Example 8 Example for prolonging the lifetime of tumor-bearing mice by AMD3100

8-week-old C57 BL/6 mice having no more than 2g of weight were taken and divided into control group, cachexia group (LLC), and AMD3100 group (LLC + AMD3100).

### Control group: no treatment.

cachexia group: 4 × 10⁶ LLC was transplanted subcutaneously into the groin of mice. After 7 days, mice were anesthetized with 100 µL of 10% chloral hydrate, and Alzet slow release pumps (model: 1004) were implanted subcutaneously on the backs of mice. The normal saline was contained in the pump.

AMD3100 group: 4 × 10⁶ LLC was transplanted subcutaneously into the groin of mice. After 7 days, mice were anesthetized with 100 µl of 10% chloral hydrate, and an Alzet slow-release pump (model: 1004) was implanted subcutaneously on its back, and AMD3100 was contained in the pump. The use amount of mice AMD3100 was 0.15mg/kg weight/day. The state of mice was continuously observed until 35 days after tumor cells were inoculated.

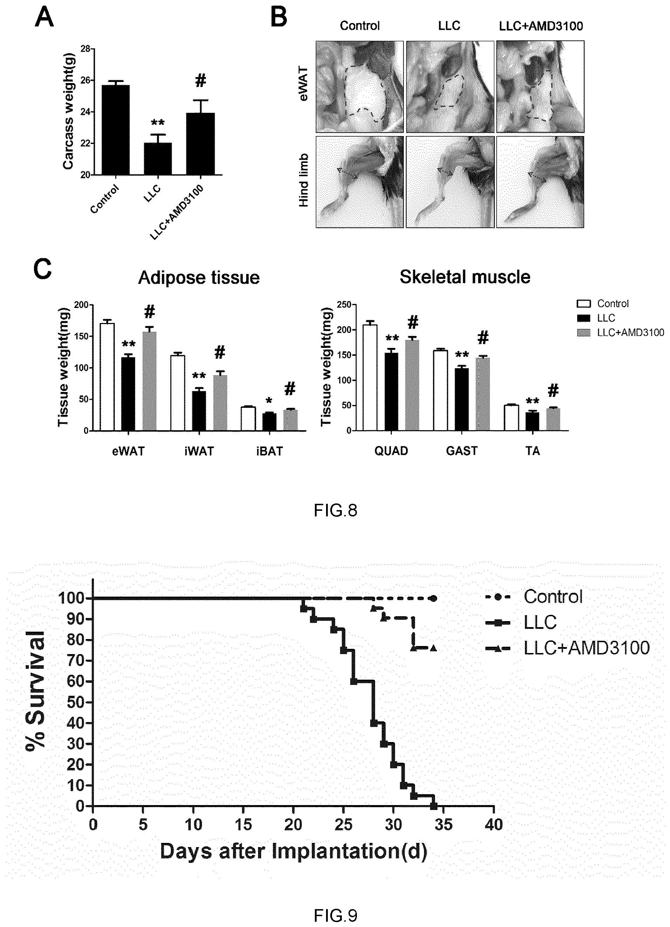

### Experiment results are seen in Fig.8 and Fig.9.

Where, Fig. A in Fig. 8 shows weights of mice in various experimental groups, and Fig. B in Fig. 8 shows atrophy of fat and muscle tissues in various experimental groups. Fig. C in Fig. 8 shows weights of fats and muscles of mice in various experimental groups. From the experimental results in Fig.8, it can be seen that after the use of AMD3100 in vivo, the symptoms of tumor cachexia in mice were relieved.

Fig. 9 is a comparison graph of lifetimes of mice in each experimental group. It can be seen that the use of AMD3100 in vivo can prolong the lifetimes of tumor-bearing mice.

Through the above experiments, it is confirmed that the occurrence of tumor cachexia is related to mesenchymal stem cells, and the content of Nestin+ mesenchymal stem cells in the muscles and fat tissues of mice with tumor cachexia is evaluated. By carrying out slicing and immunofluorescence staining on muscles and fats of mice and counting the quantity of Nestin+ cells, it is found that mice with tumor cachexia have Nestin+ cells fewer than normal mice. At the same time, fluorescence quantitative PCR also indicates that expression of Nestin in muscles and fats of mice with tumor cachexia is decreased.

The above results suggest that the occurrence of tumor cachexia is accompanied by the decrease of mesenchymal stem cells in muscles and fat tissues.

After bearing tumor to mice using Nestin-GFP transgenic mice, the inventor of the present application find Nestin GFP + cells in tumor sections. Moreover, With the occurrence of tumor cachexia, the quantity of Nestin-GFP+ cells is gradually increased. The above results suggest that the decrease of Nestin+ cells is due to the migration toward tumor, proving that the decrease of organism mesenchymal stem cells is due to the migration toward tumor.

The inventor of the present application first detects the high expression of Cxcl12 in the tumor by fluorescence quantitative PCR, and find that Nestin+ cells isolated from muscles and fat tissues of mice express Cxcr4 by immunofluorescence staining. In vitro, by using Cxcl12, the inventor finds that Transwell migration of muscle and fat-derived Nestin+ cells can be promoted. In mice, AMD3100 of 0.15mg/kg body weight/day is given to tumor-bearing mice by Alzet slow-release pump, so as to successfully inhibit the migration of Nestin+ cells from muscles and fats toward tumor so that Nestin+ cells in muscles and fats are recovered significantly compared with AMD3100 group. The above results confirm that the Cxcl12-Cxcr4 chemotaxis axis mediates the migration of mouse muscle and fat-derived Nestin+ cells.

By weighing the weights, muscles and fats of mice, the inventor of the present application finds that the cachexia symptom of mice with AMD3100 is lighter than that of mice without AMD3100 on the same days of tumor bearing. In addition, observation on the lifetime of tumor-bearing mice finds that mice with AMD3100 have lifetime longer than that of mice without AMD3100.

The above experimental results show that AMD3100 has a significant effect on the prevention and treatment of tumor cachexia. The use of AMD3100 to tumor patients can block the recruitment of tumor on mesenchymal stem cells of multiple systemic tissues such as in muscles, fats and bone marrows, is beneficial to maintaining the homeostasis of the organism tissue and delaying the occurrence of tumor cachexia, and is conductive to reducing the symptoms of cachexia in tumor patients, improving the effect of tumor treatment, improving the quality of life and prolonging the survival expectancy.

## Claims

1. Compound 1,1'-[1,4-phenylene bi(methylene)]-di-1,4,8,11-tetraazacyclotetradecane or a pharmaceutically acceptable salt thereof for use in a method of reliving and/or improving fat loss caused by cachexia.

2. The compound for use according to claim 1, wherein the cachexia is tumor cachexia.

3. A pharmaceutical composition for use in a method of relieving and/or improving fat loss caused by cachexia, the pharmaceutical composition comprising the compound 1,1'-[1,4-phenylene bis(methylene)]-di-1,4,8,11-tetraazacyclotetradecane or the pharmaceutically acceptable salt thereof.

4. The pharmaceutical composition for use in a method according to claim 3, wherein the cachexia is tumor cachexia.

## Patentansprüche

1. Verbindung 1,1'-[1,4-Phenylen-bi(methylen)]-di-1,4,8,11-tetraazacyclotetradecan oder ein pharmazeutisch akzeptables Salz derselben zur Verwendung in einem Verfahren zur Wiederherstellung und/oder Verbesserung des durch Kachexie verursachten Fettverlustes.

2. Verbindung zur Verwendung nach Anspruch 1, wobei die Kachexie eine Tumorkachexie ist.

3. Pharmazeutische Zusammensetzung zur Verwendung in einem Verfahren zur Wiederherstellung und/oder Verbesserung des durch Kachexie verursachten Fettverlustes, wobei die Zusammensetzung die Verbindung 1,1'-[1,4-Phenylen-bi(methylen)]-di-1,4,8,11-tetraazacyclotetradecan oder ein pharmazeutisch akzeptables Salz derselben enthält.

4. Pharmazeutische Zusammensetzung zur Verwendung nach Anspruch 3, wobei die Kachexie eine Tumorkachexie ist.

## Revendications

1. Composé de 1,1'-[1,4-phénylène bi(méthylène)]-di-1,4,8,11-tétraazacyclotétradécane ou un sel pharmaceutiquement acceptable de celui-ci pour son utilisation dans un procédé de soulagement et/ou d'amélioration de la perte de graisse provoquée par la cachexie.

2. Composé pour son utilisation selon la revendication 1, dans lequel la cachexie est une cachexie associée à une tumeur.

3. Composition pharmaceutique pour son utilisation dans le soulagement et/ou l'amélioration de la perte de graisse provoquée par la cachexie, la composition pharmaceutique comprenant le composé de 1,1'-[1,4-phénylène bis(méthylène)]-di-1,4,8,11-tétraazacyclotétradécane ou le sel pharmaceutiquement acceptable de celui-ci.

4. Composition pharmaceutique pour son utilisation dans un procédé selon la revendication 3, dans laquelle la cachexie est une cachexie associée à une tumeur.
